# EUROPEAN PATENT APPLICATION

(11) **EP 1 683 483 A1**
(43) Date of publication of application: **26.07.2006**
(21) Application number: 04793244.7
(22) Date of filing: 29.10.2004
(51) Int. Cl.: A61B 5/15

(54) **LANCET AND CENTESIS INSTRUMENT**

(30) Priority: 29.10.2003 JP 2003368890
(71) Applicant: Arkray, Inc., Kyoto-shi, Kyoto 601-8045 (JP)
(72) Inventor: MATSUMOTO, Daisuke, c/o ARKRAY Inc., Kyoto-shi, Kyoto 6018045 (JP); KASAI, Tokuo, c/o ARKRAY Inc., Kyoto-shi, Kyoto 6018045 (JP)
(74) Representative: Pluckrose, Anthony William
(86) International application number: PCT/JP2004/016143
(87) International publication number: WO 2005/039413

(57) **Abstract**

The present invention relates to a lancet (2) including a lancet body (20) provided with a lancing needle (20a), and a casing (22) including a space extending throughout the casing for retaining the lancet body therein. In the lancet (2), the lancet body (20) is fixed to the casing (22) when an external force exceeding a predetermined level in a particular direction is not applied to the casing (22), whereas the lancet body (20) becomes movable relative to the casing (22) when an external force exceeding the predetermined level in the particular direction is applied to the casing (22). The present invention also relates to a lancing apparatus which is used with the lancet (2) attached thereto.

## Description

### TECHNICAL FIELD

The present invention relates to a technique for extracting blood from a lancing target portion such as the skin.

### BACKGROUND ART

In a conventional method for extracting blood from skin, a disposable lancet is attached to a lancing apparatus, and the lancing needle of the lancet is caused to stick into the skin by utilizing a driving mechanism of the lancing apparatus (See Patent Document 1, for example).

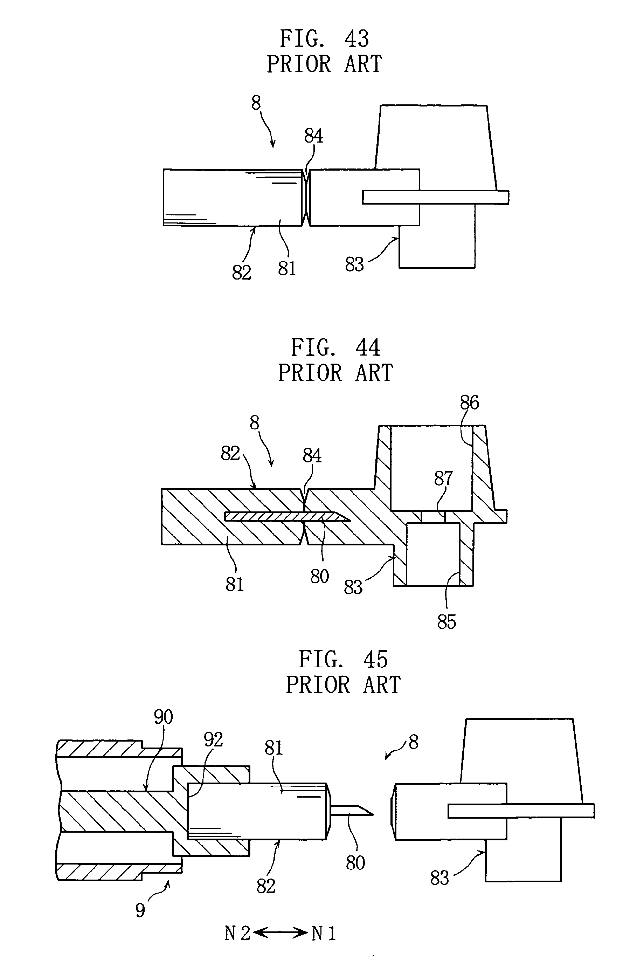

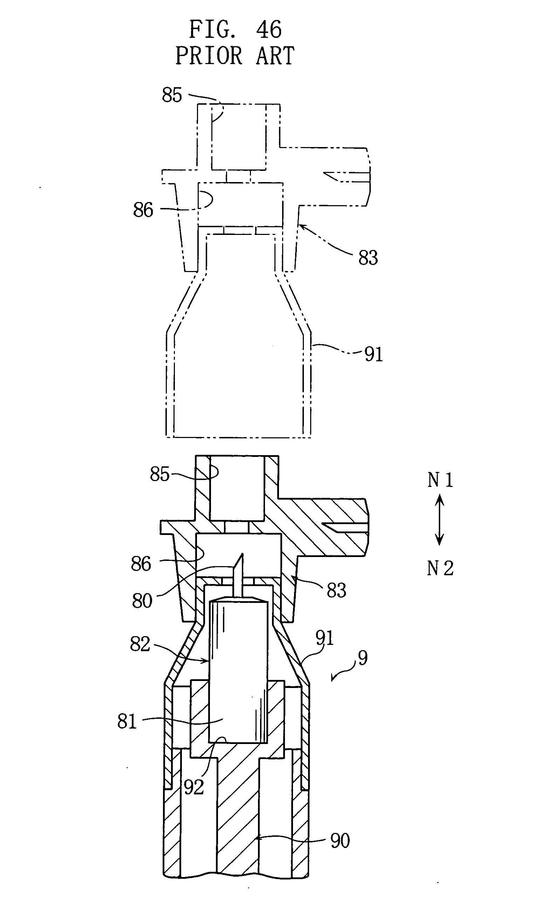

As shown in Figs. 43 and 44 of the present application, the lancet 8 disclosed in the Patent Document 1 comprises a lancet body 82 including a lancing needle 80 and a columnar portion 81, and a cap 83 covering the tip end of the lancing needle 80. A notch 84 is formed between the lancet body 82 and the cap 83. In the lancet 8, the lancet body 82 and the cap 83 are separable at the notch 84, as shown in Fig. 45. As clearly shown in Fig. 44, the cap 83 includes two recesses 85 and 86. The two recesses 85 and 86 communicate with each other via a through-hole 87. The diameter of the recess 85 corresponds to the diameter of the columnar portion 81 of the lancet body 82. As will be understood from Fig. 46, the diameter of the recess 86 corresponds to the diameter of the front end of a cover 91 of the lancing apparatus 9, which will be described later.

The lancet 8 in use is attached to the lancing apparatus 9, as shown in Figs . 45 and 46 . The illustrated lancing apparatus 9 is capable of holding the lancet 82, and includes a lancet holder 90 which is reciprocally movable in the direction indicated by N1 and N2 in the figures and the cover 91 for covering the front end of the lancet holder 90.

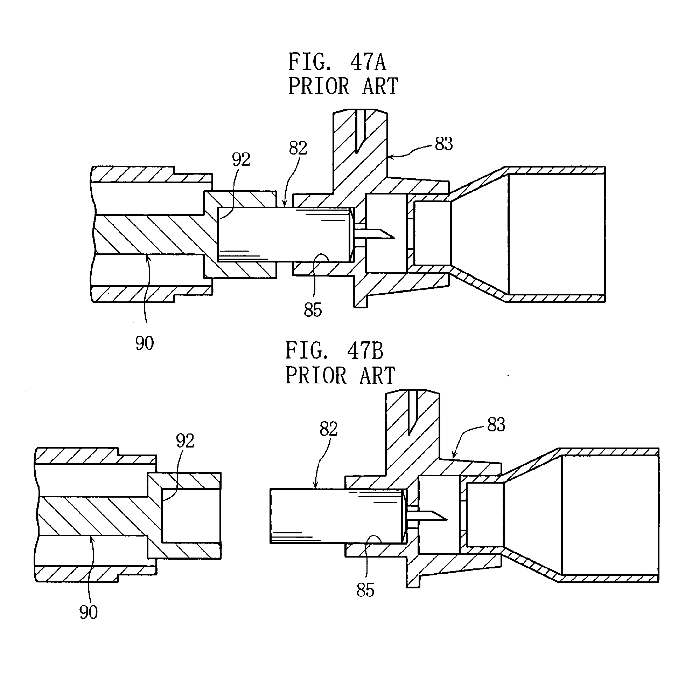

To set the lancet 8 in the lancing apparatus 9, the columnar portion 81 of the lancet body 82 is inserted into a recess 92 of the lancet holder 90, and then the cap 83 is twisted for removal from the lancet body 82. On the other hand, the detachment of the lancet 8 (lancet body 82) from the lancing apparatus 9 is performed using the cap 83 separated in the above-described manner. Specifically, as shown in Fig. 46, the recess 86 of the cap 83 is fitted to the front end of the cover 91, and then the cover 91 is removed from the lancing apparatus 9 together with the cap 83. Subsequently, as shown in Figs. 47A and 47B, the recess 85 of the cap 83 is fitted to the lancet body 82, and then the lancet body 82 is removed from the recess 92 of the lancet holder 90 together with the cap 83.

In this way, the lancet body 82 can be removed, with the lancing needle 80 of the lancet body 82 housed in the cap 83. Therefore, the possibility of touching the lancing needle 80 in disposing of the lancet 8 is reduced, which is advantageous in terms of hygiene. However, the lancing needle 80 is exposed when the lancet body 82 and the cap 83 are separated as shown in Fig. 45 and also when the cover 91 is removed to detach the lancet body 82 from the lancet holder 90. Therefore, in the lancet 8, the lancing needle 80 is exposed in attaching the lancet body 82 to the lancet holder 90 as well as in detaching the lancet body 82 from the lancet holder 90, which is dangerous. Moreover, the detachment of the lancet body 82 by using the cap 83 is complicated and sometimes difficult particularly for elderly people or people with weak eyesight.

Patent Document 1: JP-A 5-285127

### DISCLOSURE OF THE INVENTION

An object of the present invention is to make it possible to attach and remove a lancet by an easy operation and to dispose of a lancet hygienically.

According to a first aspect of the present invention, there is provided a lancet comprising a lancet body provided with a lancing needle, and a casing including a space extending throughout the casing for retaining the lancet body therein. The lancet body is fixed to the casing when an external force exceeding a predetermined level in a particular direction is not applied to the casing, whereas the lancet body becomes movable relative to the casing when an external force exceeding the predetermined level in the particular direction is applied to the casing.

For instance, the particular direction crosses the direction in which the space extends, and typically, the particular direction is perpendicular to the direction in which the space extends.

For instance, the casing has a cross-sectional shape which changes when the external force exceeding the predetermined level in the particular direction is applied to the casing. In this instance, the lancet body becomes movable relative to the casing when the cross-sectional shape of the casing is changed.

For instance, the casing includes a contact portion which comes into contact with the lancet body when the external force exceeding the predetermined level in the particular direction is not applied to the casing. In this case, a gap is defined between the contact portion and the lancet body when the cross-sectional shape of the casing is changed. The contact portion may comprise a projection. Alternatively, the contact portion may comprise a recess. In this instance, the lancet body is provided with a projection for coming into engagement with the recess. The outer diameter of the casing at a portion where the contact portion is not provided may be larger than the outer diameter of the casing at a portion where the contact portion is provided.

Preferably, the casing may be formed with a projection for actively causing the external force in the particular direction to be applied to the casing. The casing may be formed with an opening for allowing the cross-sectional shape of the casing to change when the external force in the particular direction is applied. For instance, the opening may comprise a cutout or a slit.

Preferably, after the cross-sectional shape of the casing is changed, the cross-sectional shape returns to its original shape when the application of the external force to the casing in the particular direction is removed.

The lancet according to the present invention may further comprise a lid for selectively opening or closing an upper opening of the casing. For instance, the lid is attached to the casing. The lid may be separate from the casing.

When the lid, if it is provided at the lancet, closes the upper opening of the casing, the external force exceeding the predetermined level in the particular direction may be applied to the casing to change the cross-sectional shape of the casing. In this instance, the lancet body becomes movable relative to the casing when the cross-sectional shape of the casing is changed. Further, the casing may be formed with an opening for allowing the cross-sectional shape of the casing to change when the external force in the particular direction is applied. In this instance, the lid may include an operative portion which comes into engagement with the opening and applies the external force in the particular direction to the casing when the lid closes the upper opening. Preferably, the casing includes a stopper portion for preventing the lancet body from dropping through a lower opening of the casing when the lancet body is movable relative to the casing. Preferably, the lancet including a lid may further comprise a fixer for fixing the lid to the casing when the upper opening is closed by the lid. For instance, the fixer comprises a projection provided at one of the lid and the casing, and a hook provided at the other one of the lid and the casing for engagement with the projection.

The casing may hold an analytical tool for analyzing a particular component contained in body fluid extracted from a lancing target portion. When the lancet includes a lid, the analytical tool may be provided at the lid. For instance, the analytical tool includes a capillary for moving blood by capillary force, a through-hole for allowing movement of the lancing element, and an introduction port which communicates with the through-hole for introducing blood to the capillary.

According to a second aspect of the present invention, there is provided a lancing apparatus for use with a lancet attached to the lancing apparatus. The lancet to be used may include a lancet body provided with a lancing needle, and a casing including a space extending throughout the casing for retaining the lancet body therein, and the lancet body may be fixed to the casing when an external force exceeding a predetermined level in a particular direction is not applied to the casing, whereas the lancet body may become movable relative to the casing when an external force exceeding the predetermined level in the particular direction is applied to the casing. In such a case, the lancing apparatus is designed to apply an external force to the casing to make the lancet body movable relative to the casing once the lancet is set.

For instance, the lancing apparatus comprises an operative portion for coming into engagement with the casing to apply an external force to the casing.

When the casing of the lancet to be used is formed with a projection for actively causing the external force in the particular direction to be exerted to the casing, the operative portion may be designed to come into engagement with the projection to apply the external force to the casing once the lancet is set.

When the casing of the lancet to be used is formed with an opening for allowing the cross-sectional shape of the casing to change when the external force in the particular direction is applied to the casing, the operative portion is designed to come into engagement with the opening to change the cross-sectional shape of the casing when the lancet is set.

According to a third aspect of the present invention, there is provided a lancing apparatus for use with a lancet attached to the lancing apparatus. The lancet to be used may include a lancet body provided with a lancing needle, a casing including a space extending throughout the casing for retaining the lancet body therein and a lid for closing an upper opening of the casing, and the lancet body may be fixed to the casing when an external force exceeding a predetermined level in a particular direction is not applied to the casing, whereas the lancet body may become movable relative to the casing when an external force exceeding the predetermined level in the particular direction is applied to the casing by closing the upper opening by the lid. In such a case, the apparatus comprises external force applying means for applying an external force to the lid to close the upper opening by the lid in a state in which the lancet is attached.

For instance, the lancing apparatus of the present invention may comprise an accommodation space for accommodating the lancet. In this instance, the external force applying means includes a slider which is reciprocally movable in a direction crossing (typically perpendicular to) the direction in which the accommodation space extends in a state in which the lancet is accommodated in the accommodation space. The slider includes an engagement portion for coming into engagement with the lid.

For instance, the engagement portion is reciprocally movable between a first position directly above the accommodation space and a second position avoiding a position directly above the accommodation space. The engagement portion, in moving from the second position to the first position, comes into engagement with the lid to apply an external force to the lid to cause the lid to close an upper opening of the casing.

When the casing of the lancet to be used includes a stopper portion for preventing the lancet body from dropping through a lower opening of the casing in a state in which the lancet body is movable relative to the casing, the lancing apparatus further comprises a lancet holder for holding the lancet body. For instance, the lancet holder may be movable without interfering with the stopper portion in a state in which the lancet is attached. In this instance, the lancet holder may be formed with a recess for avoiding the interference with the stopper portion.

### BRIEF DESCRIPTION OF THE DRAWINGS

Fig. 1 is a front view showing a lancing apparatus according to a first embodiment of the present invention.
Fig. 2 is a sectional view of the lancing apparatus shown in Fig. 1.
Fig. 3 is an enlarged sectional view for describing the lancing mechanism of the lancing apparatus shown in Fig. 1.
Fig. 4 is an enlarged sectional view for describing the liquid supplying mechanism of the lancing apparatus shown in Fig. 1.
Fig. 5 is a perspective view illustrating a lid of the lancing apparatus of Fig. 1 by seeing through the main body of the lid.
Fig. 6 is a sectional view taken along lines VI-VI in Fig. 4.
Fig. 7 is a sectional view taken along lines VII-VII in Fig. 4.
Fig. 8 is an overall perspective view of a lancet according to the present invention which is used in the lancing apparatus shown in Fig. 1.
Fig. 9. is a sectional view taken along lines IX-IX in Fig. 8.
Fig. 10 is a sectional view taken along lines X-X in Fig. 8.
Fig. 11 is a sectional view of the lancet shown in Figs. 8-10 in the state in which the cap is removed.
Fig. 12 is a sectional view taken along lines XII-XII in Fig. 8.
Fig. 13 is a partially cut-away perspective view for describing the internal structure of the lancet.
Fig. 14 is a plan view of the lancet shown in Fig. 13.
Fig. 15 is a sectional view for describing the operation of the lancet.
Fig. 16 is a sectional view for describing the operation of the lancet.
Fig. 17 is an overall perspective view of a biosensor incorporated in the lancet.
Fig. 18 is an exploded perspective view of the biosensor shown in Fig. 17.
Fig. 19 is a sectional view taken along lines XIX-XIX in Fig. 17.
Fig. 20 is a sectional view taken along lines XX-XX in Fig. 17.
Fig. 21 includes sectional views of a principal portion for describing the use of the lancing apparatus shown in Fig. 1.
Fig. 22 includes sectional views of a principal portion for describing the operation of the lancing mechanism.
Fig. 23 includes sectional views of a principal portion for describing the process of introducing blood in the biosensor.
Fig. 24 is an overall perspective view showing a lancet according to a second embodiment of the present invention.
Fig. 25 is a plan view of the lancet shown in Fig. 24.
Fig. 26 is a sectional view taken along lines XXVI-XXVI in Fig. 24 showing the state in which the lancet of Fig. 24 is attached to a housing.
Fig. 27A is a plan view for describing the operation of the lancet shown Fig. 24, whereas Fig. 27B is a sectional view corresponding to Fig. 26 for describing the operation of the lancet shown Fig. 24.
Fig. 28 is a sectional view showing another example of lancet.
Fig. 29 is a sectional view showing still another example of lancet.
Fig. 30 is a sectional view taken along lines XXX-XXX in Fig. 29 showing the state in which the lancet of Fig. 29 is set in a housing for describing the operation of the lancet.
Fig. 31 is a sectional view showing still another example of lancet.
Fig. 32 is a front view showing a lancing apparatus according to a third embodiment of the present invention.
Fig. 33 is a sectional view of the lancing apparatus shown in Fig. 32.
Fig. 34 is a perspective view of a principal portion of the lancing apparatus shown in Fig. 32.
Fig. 35 is an overall perspective view of a lancet holder of the lancing apparatus shown in Fig. 32.
Fig. 36A is an overall perspective view of a lancet according to the present invention used in the lancing apparatus shown in Fig. 32, whereas Fig. 36B is a plan view of a casing of the lancet.
Fig. 37 includes sectional views of the lancet shown in Fig. 36.
Fig. 38 is a sectional view for describing the operation of the lancet shown in Fig. 36.
Fig. 39A is a front view for describing the operation of the lancet shown in Fig. 36, whereas Fig. 39B is a sectional view thereof.
Fig. 40 is a sectional view for describing the operation of the lancing apparatus shown in Fig. 32.
Fig. 41 is a sectional view for describing the operation of the lancing apparatus shown in Fig. 32.
Fig. 42 is an enlarged sectional view of a principal portion of the lancing apparatus shown in Fig. 32 for describing the operation of the lancing apparatus.
Fig. 43 is a front view showing an example of prior art lancet.
Fig. 44 is a sectional view of the lancet shown in Fig. 43.
Fig. 45 is a front view, partially in section, for describing the operation of attaching the lancet shown in Figs. 43 and 44 to a lancing apparatus.
Fig. 46 is a sectional view for describing the operation to remove a lancet from a lancing apparatus.
Fig. 47 is a sectional view for describing the operation to remove a lancet from a lancing apparatus.

### BEST MODE FOR CARRYING OUT THE INVENTION

A first through a third embodiments of the present invention will be described below.

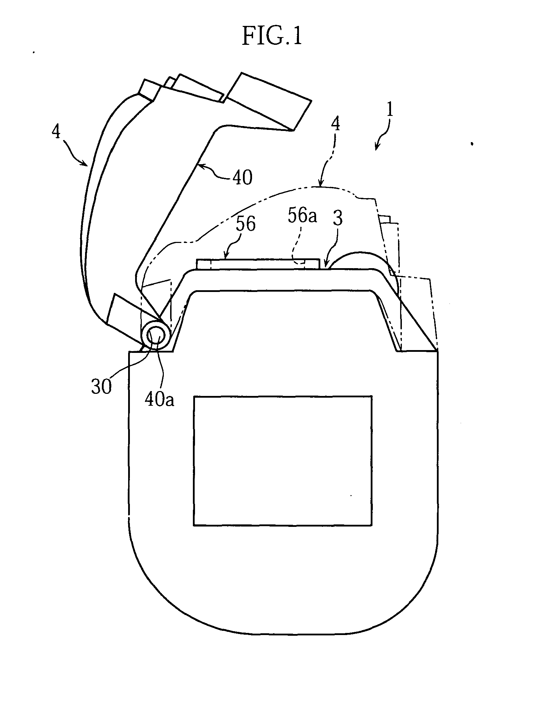

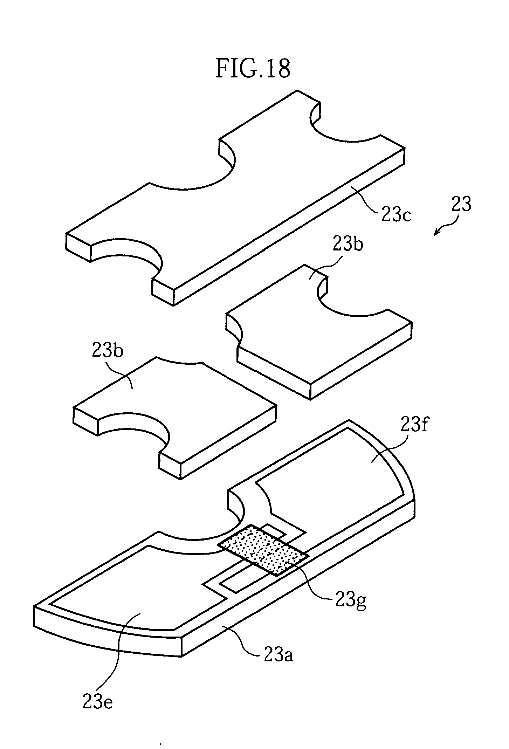

Firstly, the first embodiment of the present invention will be described with reference to Figs. 1-23.

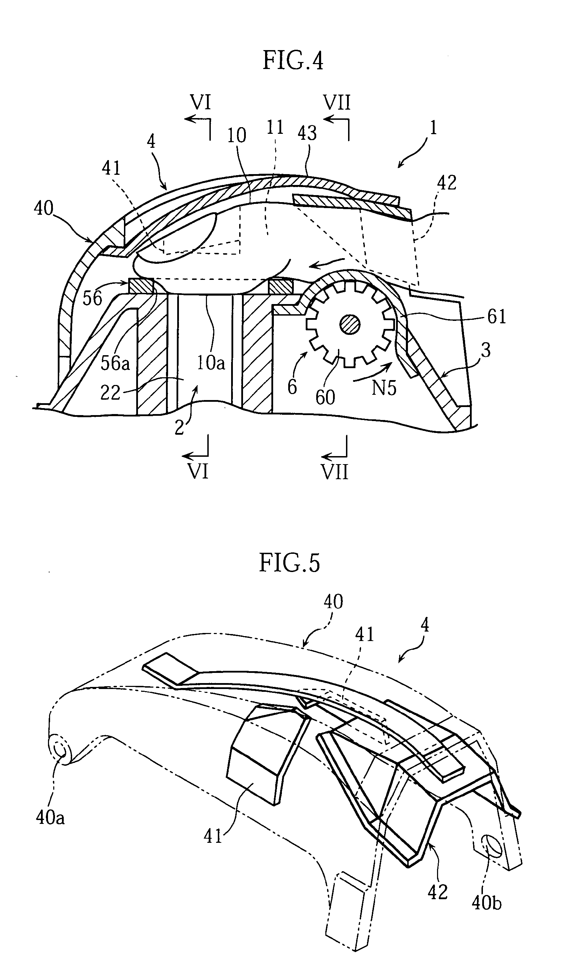

Figs. 1-3 show a lancing apparatus 1 which is used with a lancet 2 attached thereto. The lancing apparatus includes an apparatus body 3, and a lid 4 which defines an accommodation space 11 (See Fig. 4) for inserting a fingertip 10 between the apparatus body 3 and the lid.

The apparatus body 3 includes a lancing mechanism 5 and a liquid supplying mechanism 6.

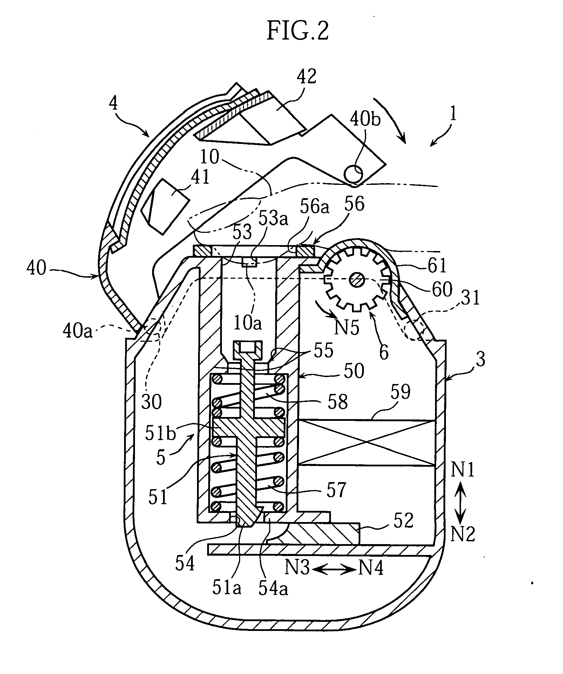

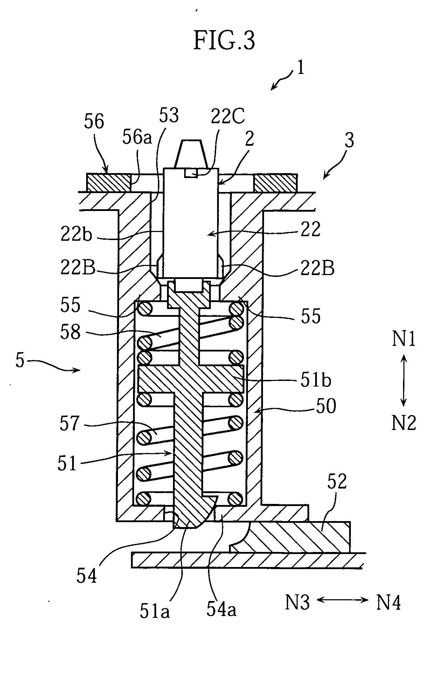

As shown in Figs. 2 and 3, the lancing mechanism 5 serves to lance the fingertip 10 (See Fig. 4) retained in the accommodation space 11, and includes a housing 50, a lancet holder 51 and an operative portion 52.

The housing 50 serves to accommodate the lancet 2 and the lancet holder 51. The housing 50 includes an opening 53, a through-hole 54 and a pair of stepped portions 55.

The opening 53, which is utilized for inserting the lancet 2, includes a cutout 53a. The cutout 53a serves to engage with a projection 22c of the lancet 2, which will be described later. A contact member 56 is arranged on the opening 53. The contact member 56 is brought into close contact with the fingertip 10 when the fingertip is inserted into the accommodation space 11 (See Fig. 4). The contact member 56 is formed with a through-hole 56a. The through-hole 56a is larger in diameter than the opening 53 of the housing 50. The contact member 56 is made of an elastic body such as rubber or a foamed material to readily come into close contact with the fingertip 10.

The through-hole 54 serves to allow the movement of an engagement portion 51a of the lancet holder 51. The periphery 54a of the through-hole 54 serves to engage with the engagement portion 51a.

The paired stepped portions 55 serve to apply a pressing force to a casing 22 of the lancet 2, which will be described later, and project toward the inside of the housing 50. Each of the paired stepped portions 55 is tapered on the upper side (N1 side) in Figs. 2 and 3.

The lancet holder 51 serves to hold the lancet 2. The lancet holder 51 is provided with the engagement portion 51a and a flange portion 51b. The engagement portion 51a is in the form of a hook which is capable of engaging the periphery 54a of the through-hole 54 of the housing 50. A coil spring 57 is arranged between the flange portion 51b and the periphery 54a of the through-hole 54. The coil spring 57 is compressed when the engagement portion 51a is brought into engagement with the periphery 54a of the through-hole 54. Therefore, when the engagement portion 51a is disengaged from the periphery 54a of the through-hole 54, the elastic restoring force of the coil spring 57 is exerted to the flange portion 51 to move the lancet holder 51 in the direction indicated by arrow N1. A coil spring 58 is arranged between the flange portion 51b and the stepped portions 55. The coil spring 58 is compressed when the lancet holder 51 is moved in the direction of arrow N1, and the elastic restoring force can be exerted to the flange portion 51b to move the lancet holder 51 in the direction indicated by arrow N2. The coil spring 58 can be dispensed with.

The operative portion 52 serves to disengage the engagement portion 51a of the lancet holder 51 from the periphery 54a of the through-hole 54 and is movable in the directions indicated by arrows N3 and N4. Specifically, when the operative portion 52 is moved in the direction of arrow N3, the operative portion engages the engagement portion 51a to exert a pressing force to the engagement portion 51a so that the engagement portion 51a is disengaged from the periphery 54a of the through-hole 54. The movement of the operative portion 52 in the directions of arrows N3 and N4 is controlled by non-illustrated control means. For instance, in conjunction with the open/close operation of the lid 4, the control means moves the operative portion 52 in the direction of arrow N3 by utilizing an actuator 59 to disengage the engagement portion 51a. Alternatively, the operative portion 52 may be moved manually.

As shown in Figs. 2 and 4, the liquid supplying mechanism 6 serves to supply blood from the rood side of the fingertip 10 toward a lancing target portion 10a of the fingertip. The liquid supplying mechanism 6 includes a rotating member 60 comprising a gear. The rotating member 60 is rotatable in the direction of arrow N5 and so arranged as to come into contact with the fingertip 10 accommodated in the accommodation space 11 via a sheet member 61.

As shown in Figs. 4 and 5, the lid 4 includes a main body 40, and a pair of compressing portions 41 and a binding portion 42 which are supported by the main body.

The main body 40 defines the accommodation space 11 for inserting the fingertip between the main body and the apparatus body 3 and is capable of selectively covering the through-hole 56a of the contact member 56 of the apparatus body 3 (See Fig. 1). The main body 40 is open downward to cover the apparatus body 3 and also open at one side for allowing the insertion of a fingertip into the accommodation space 11. As shown in Figs. 1 and 2, a shaft portion 40a is provided at an end of the main body 40. The shaft portion 40a is fitted in a through-hole 30 (See Fig. 2) of the apparatus body 3, and the main body 40 is pivotable around the shaft portion 40a relative to the apparatus body 3. The main body 40 is further formed with a recess 40b. When the lid 4 covers the apparatus body 3, the recess 40b engages a projection 31 of the apparatus body 3 to maintain the closed state of the lid 4. In this state, the accommodation space 11 is open at one side, as shown in Fig. 4.

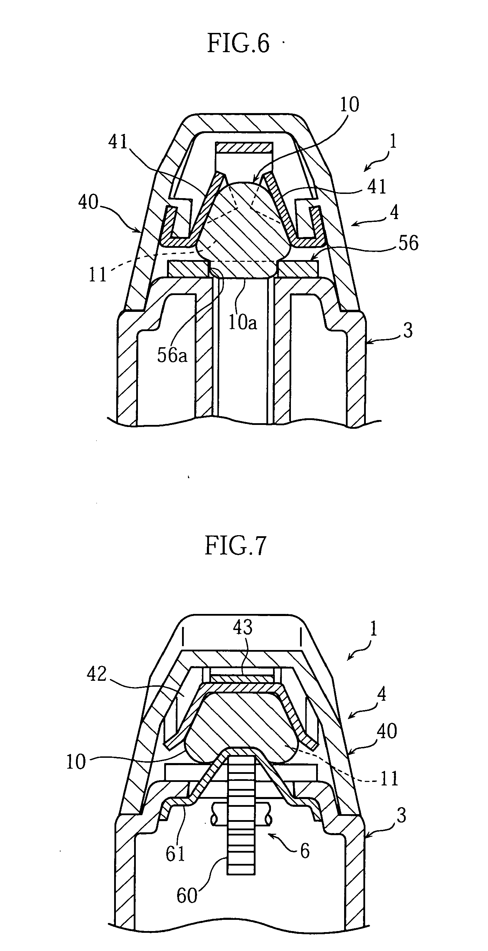

As shown in Figs. 4 and 6, when the fingertip 10 is inserted into the accommodation space 11, the paired compressing portions 41 press the lancing target portion 10a of the fingertip 10 against the through-hole 56a of the contact member 56 of the apparatus body 3. Each of the compressing portions 41 is so fixed to an inner surface of the main body 40 as to be located directly above the contact member 56 when the main body 40 covers the apparatus body 3. The outer surfaces of the two compressing portions 41 are configured to conform to the outer configuration of the fingertip 10.

As shown in Figs. 4 and 7, the binding portion 42 serves to bind a portion of the fingertip on the root side of the lancing target portion 10a to congest the fingertip 10. The binding portion 42 is supported by the main body 40 via a leaf spring 43 and is swayable relative to the main body 40. When the fingertip 10 is inserted in the accommodation space 11, the binding portion 42 faces the rotating member 60 of the liquid supplying mechanism 6. Therefore, the spring force of the leaf spring 43 is exerted, via the binding portion 42, to the root portion of the fingertip in the accommodation space 11, whereby the root portion of the finger is pressed against the rotating member 60. In this way, the fingertip 10 is congested by the binding portion 42.

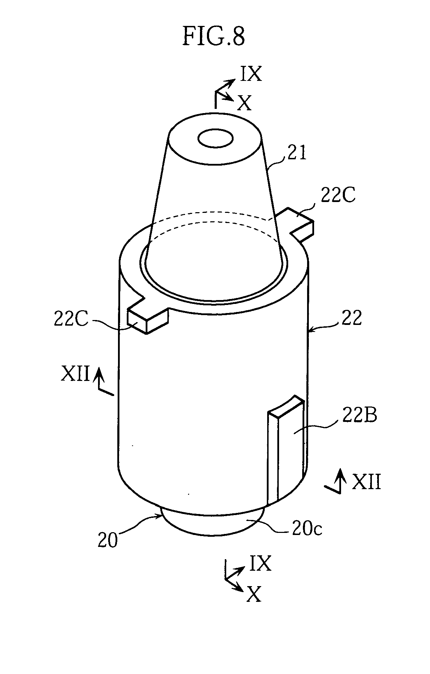

As shown in Fig. 3, the lancet 2, including an element to lance a fingertip, is attached in use to the housing 50 of the lancing apparatus 3. As shown in Figs. 8-10, the lancet 2 includes a lancet body 20, a cap 21, a casing 22 and a biosensor 23.

The lancet body 20 includes a lancing needle 20a, a protector 20b for covering the tip end of the lancing needle 20a, and a holding portion 20c in which an end of the lancing needle 20a which is opposite from the tip end is embedded. A cutout 20d is formed between the protector 20b and the holding portion 20c so that the protector 20b and the holding portion 20c are separable at the cutout 20d. The protector 20b is formed with a recess 20e. The recess 20e serves to receive an engagement support portion 21a of the cap 21, which will be described later.

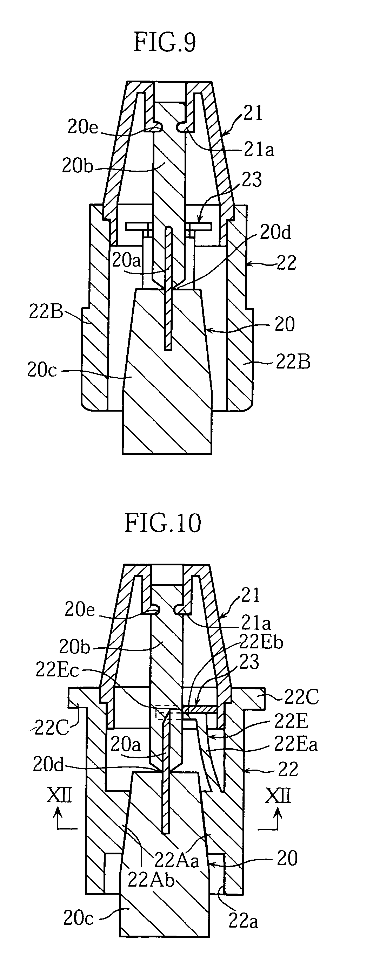

As shown in Figs. 9-11, the cap 21 is removably attached to the casing 22 and holds the protector 20b of the lancet body 20 at the engagement support portion 21a. Therefore, by detaching the cap 21 from the casing 22, the protector 20b of the lancet body 20 can be removed from the lancing needle 20a to expose the lancing needle.

As shown in Figs. 8-10, the casing 22 holds the lancet body 20 and the biosensor 23 and is cylindrical as a whole. The casing 22 includes three pairs of projections 22Aa, 22Ab, 22B and 22C.

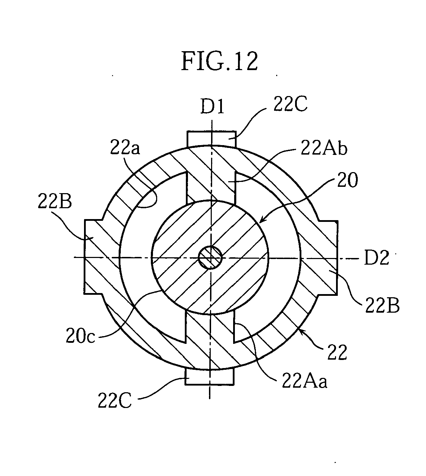

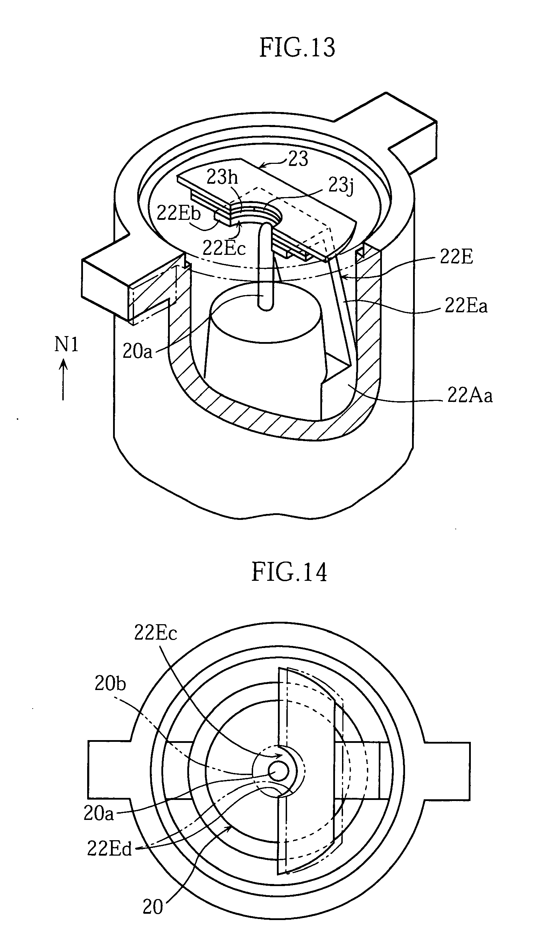

As shown in Figs. 10 and 12, the paired projections 22Aa and 22Ab are portions to come into close contact with the holding portion 20c of the lancet body 20 and project from the inner surface 22a of the casing 22 in a direction along a first diameter axis D1. As better shown in Figs. 10 and 13, a supporter 22E for supporting the biosensor 23 extends from the projection 22Aa. The supporter 22E includes a spring portion 22Ea serving as a leaf spring and a support portion 22Eb on which the biosensor 23 is to be placed. The support portion 22Eb is formed with a cutout 22Ec. As indicated by phantom lines in Fig. 14, with the support portion 22Eb positioned slightly outward relative to the natural state, the inner surface 22Ed of the cutout 22Ec engages the protector 20b of the lancet body 20. When the lancing needle 20a is exposed by removing the protector 20b of the lancet body 20, the inner surface 22Ed of the cutout 22Ec comes to a position in the natural state. In this natural state, a gap is defined between the inner surface 22Ed of the cutout 22Ec and the lancing needle 20a.

As shown in Figs. 3 and 9, the paired projections 22B serve to engage the stepped portions 55 of the housing 50 of the apparatus body 3 and are provided at a lower portion of the outer surface 22b of the casing 22 at positions along a second diameter axis D2 which is perpendicular to the first diameter axis D1, as shown in Fig. 12.

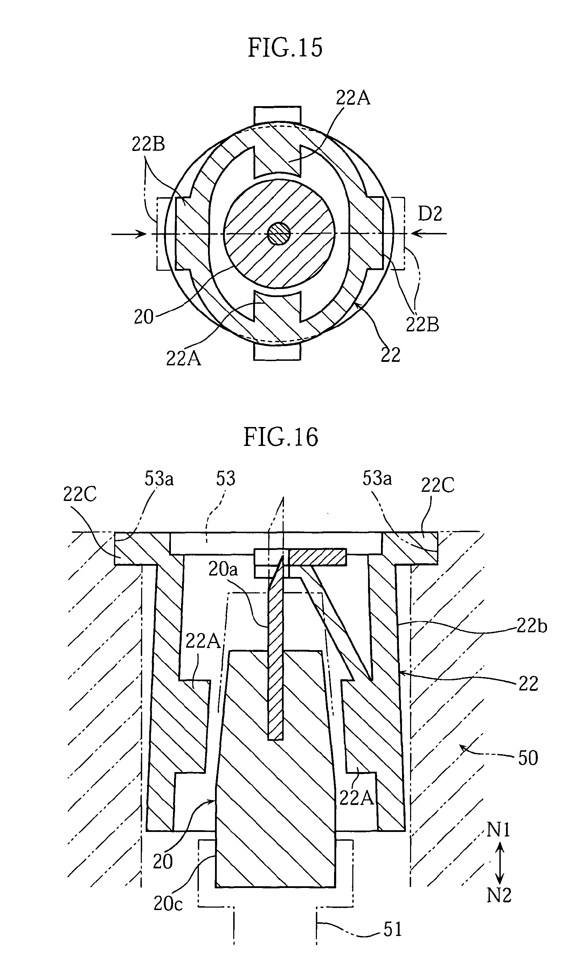

As shown in Figs. 12 and 16, the paired projections 22C are provided at an upper portion of the outer surface 22b of the casing 22 at positions on the first diameter axis D1. In inserting the lancet 2 into the housing 50 of the apparatus body 3, the projections 22C engage the cutout 53a of the opening 53. Thus, the projections 22C are utilized for positioning the lancet 2 in inserting the lancet into the housing 50.

The casing 22 having the above-described structure is formed by e.g. resin molding so that the entirety is elastically deformable. Therefore, as shown in Figs. 15 and 16, when an inward force is applied to the projections 22B along the second diameter axis D2, the casing 22 deforms at least at the portions provided with the projections 22B. Therefore, the distance between the projections 22B decreases, whereas the distance between the projections 22A increases, whereby a gap is defined between the projections 22A and the lancet body 20. As a result, the lancet body 20 becomes movable relative to the casing 22 in the directions of arrows N1 and N2.

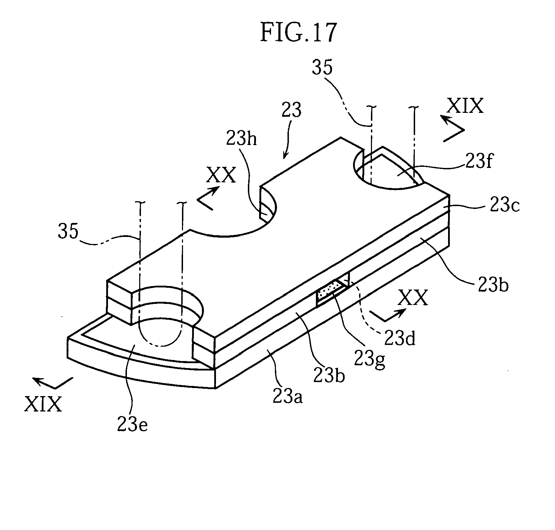

The biosensor 23 shown in Figs. 17-20 is designed to move blood by capillary force and measure the concentration of a particular component (e.g. glucose) in blood by an electrode method. Specifically, the biosensor 23 comprises a substrate 23a, a pair of spacers 23b and a cover 23c, and includes a capillary 23d defined by the elements 23a-23c.

The substrate 23a has an obverse surface formed with a counter electrode 23e, a working electrode 23f and a reagent portion 23g. As will be understood from Fig. 17, the counter electrode 23e and the working electrode 23f are to be electrically connected to an analysis circuit (not shown) in the apparatus body 3 via terminals 35. The reagent portion 23g is so formed as to connect the working electrode 23e and the counter electrode 23f to each other and includes oxidoreductase and electron mediator, for example.

The paired spacers 23b serve to define the width and height of the capillary 23d and are spaced from each other across the reagent portion 23g on the obverse surface of the substrate 23a. The spacers 23b may comprise a double-sided tape, for example.

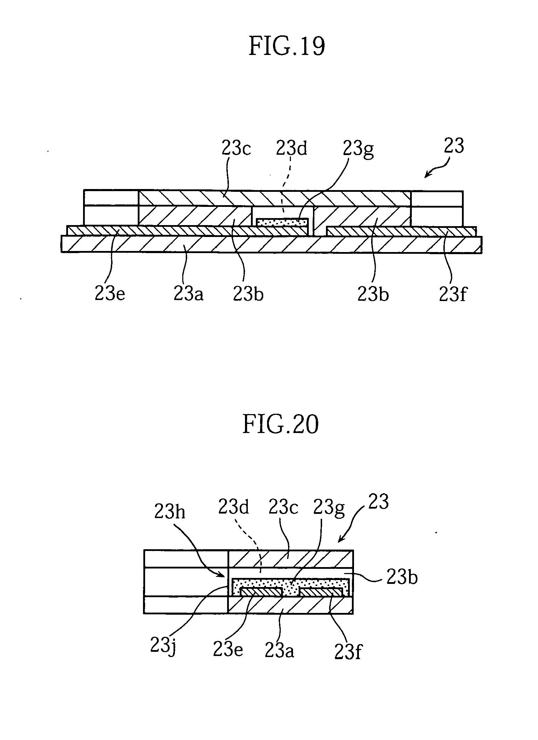

As better shown in Figs. 13 and 20, the biosensor 23 is formed with a semicircular cutout 23h. The cutout 23h allows the movement of the lancing needle 20a when the lancet body 20 is moved in the direction of arrow N1. The cutout 23h communicates with an opening 23j for introducing blood into the capillary 23d. The biosensor 23 is fixed to the support portion 22Eb so that the cutout 23h corresponds to the cutout 22Ec of the support portion 22Eb. Therefore, when the support portion 22Eb is in the natural state, a gap is defined between the cutout 23h and the lancing needle 20a.

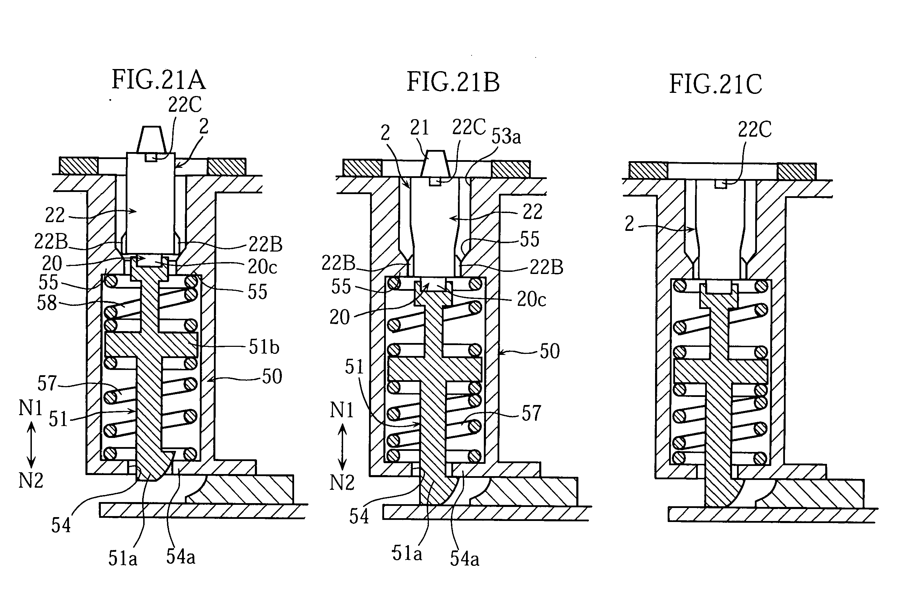

As shown in Figs. 2 and 3, to perform the lancing operation using the lancing apparatus 1, the lid 4 is opened, and the lancet 2 is inserted into the housing 50 via the through-hole 56a of the contact member 56, as shown in Figs. 2 and 3. As shown in Figs. 21A and 21B, the insertion of the lancet 2 can be performed by pushing the lancet 2 into the housing 50 while positioning the projections 22C of the lancet 2 at the cutout 53a of the housing 50 (See Fig. 16).

When the lancet 2 is pushed into the housing 50, the holding portion 20c of the lancet body 20 is fitted into the lancet holder 51, while the projections 22B of the casing 22 engage the stepped portions 55 of the housing 50. As a result, as shown in Figs. 15 and 16, the casing 22 deforms to cause the lancet body 20 to be movable relative to the casing 22. In this state, since the holding portion 20c of the lancet body 20 is fitted in the lancet holder 51, the lancet body 20 can move together with the lancet holder 51. In inserting the lancet 2 into the housing 50, the lancet holder 51 can be moved in the direction of arrow N2 to cause the engagement portion 51a of the lancet holder 51 to engage the periphery 54a of the through-hole 54 of the housing 50. By this operation, the coil spring 57 is compressed and stores elastic restoring force. Alternatively, the engagement portion 51a of the lancet holder 51 may be brought into engagement with the periphery 54a of the through-hole 54 by an operation which is separate from the operation to set the lancet 2. Specifically, the engagement portion 51a may be brought into engagement with the periphery 54a of the through-hole 54 before the lancet 2 is set to the lancet holder 51.

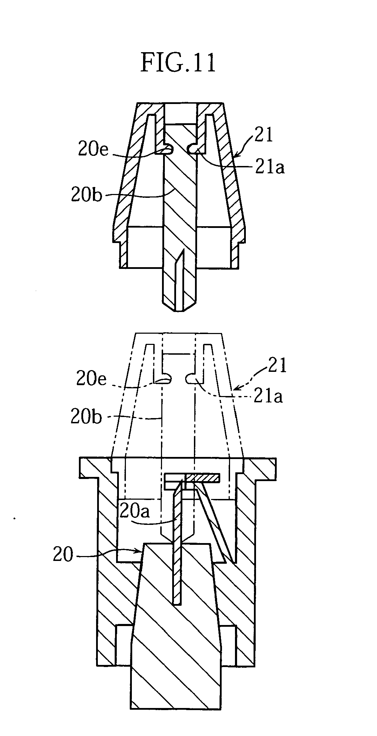

Subsequently, as shown in Figs. 11, 21B and 21C, the cap 21 is removed from the lancet 2. As better shown in Fig. 11, the engagement support portion 21a of the cap 21 is fitted in the recess 20e of the protector 20b of the lancet body 20. Therefore, in removing the cap 21, the protector 20b is also pulled out from the lancet 2. As a result, the tip end of the lancing needle 20a is exposed. As shown in Fig. 14, by this operation, the biosensor 23 moves to a position where a slight gap is defined between the cutout 23h, i.e., the opening 23j and the tip end of the lancing needle 20a. As will be understood from Fig. 17, the working electrode 23e and the counter electrode 23f of the biosensor 23 come into contact with the terminals 35 provided at the apparatus body 3. In this state, a voltage can be applied across the working electrode 23e and the counter electrode 23f, and the amount of electrons supplied to the working electrode 23e can be measured as the electric current.

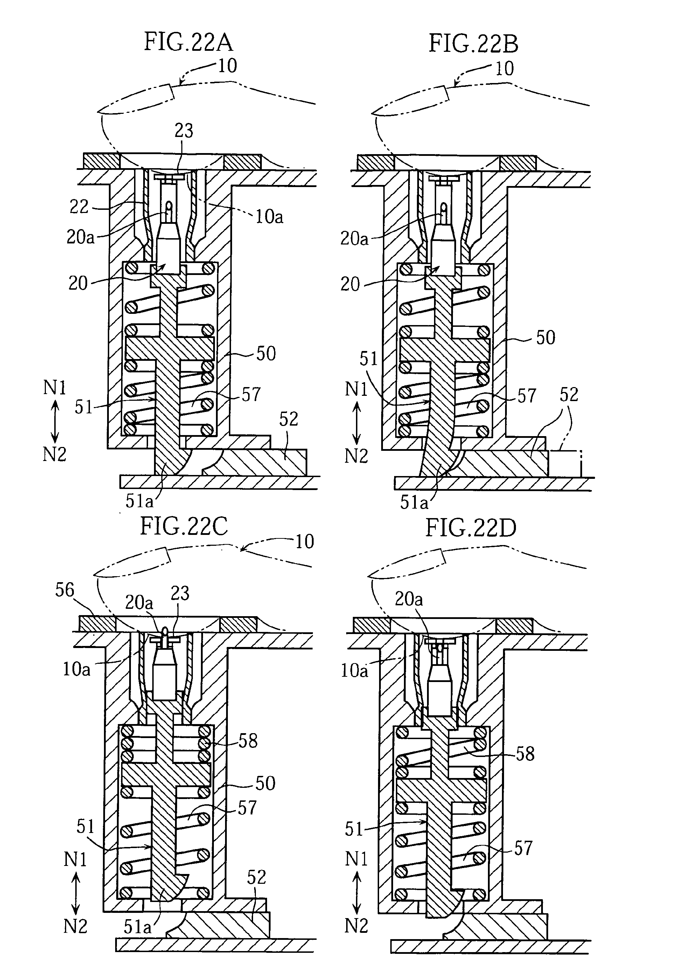

Subsequently, as shown in Fig. 4, the fingertip 10 is placed on the apparatus body 3 so as to close the through-hole 56a of the contact member 56, and then the lid 4 is closed. The closed state of the lid 4 is maintained by bringing the projection 31 of the apparatus body 3 into engagement with the recess 40b of the lid 4 (See Fig. 2). In this state, the accommodation space 11 is defined between the lid 4 and the apparatus body 3, and the fingertip 10 is held in the accommodation space 11. As shown in Figs. 4 and 6, in the accommodation space 11, the compressing portions 41 press the portion of the fingertip 10 around the lancing target portion 10a against the contact member 56. Therefore, the lancing target portion 10a bulges out from the through-hole 56a of the contact member 56 and enters the casing 22 of the lancet 2. Therefore, the lancing target portion 10a is congested so that blood is accumulated to the lancing target portion 10a, and the lancing target portion 10a comes into contact with the biosensor 23 in the casing 22, as shown in Fig. 22A. As shown in Figs. 4 and 7, in the accommodation space 11, the binding portion 42 presses the root portion of the fingertip against the rotating member 60. In this state, the fingertip 10 is congested, and blood is kept accumulated at the fingertip 10.

Subsequently, as shown in Figs. 22A-22C, the lancet body 20 is moved in the direction of arrow N1 to stick the lancing target portion 10a of the fingertip 10 with the lancing needle 20a. The movement of the lancet body 20 is performed by moving the operative portion 52 to come into contact with the engagement portion 51a and disengaging the lancet holder 51 from the housing 50. When the lancet holder 51 is disengaged, the lancet holder 51 together with the lancet body 20 moves in the direction of arrow N1 due to the elastic restoring force of the coil spring 57, so that the lancing needle 20a of the lancet body 20 sticks into the lancing target portion 10a.

As shown in Fig. 22C, when the lancing needle 20a sticks into the lancing target portion 10a, the lancing target portion 10a of the fingertip 10 is cut and bleeds. Since a slight gap is defined between the lancing needle 20a and the opening 23j of the biosensor 23, the biosensor 23 does not hinder the bleeding. Since blood is accumulated to the fingertip 10 and hence to the lancing target portion 10a by the action of the compressing portions 41, the binding member 42 (See Figs. 4, 6 and 7) and the contact member 56, blood is readily extracted from the lancing target portion 10a.

As shown in Figs. 22C and 22D, after the lancing needle 20a is stuck into the lancing target portion 10a, the lancet holder 51 retreats due to the elastic restoring force of the coil springs 57 and 58, so that the lancing needle 20a is immediately pulled out from the fingertip 10. At the same time, the rotating member 60 shown in Fig. 4 is rotated in the direction of arrow N5. By this rotation, the root portion of the finger is rubbed toward the fingertip 10 via the sheet member 61, whereby blood is accumulated to the fingertip 10. This operation of the rotating member 60 also contributes to the proper bleeding from the lancing target portion 10a. The rotation of the rotating member 60 need not necessarily be performed after the lancing needle 20a is stuck into the lancing target portion 10a. The rotation may be performed before the sticking of the lancing needle 20a or successively before and after the sticking of the lancing needle 20a.

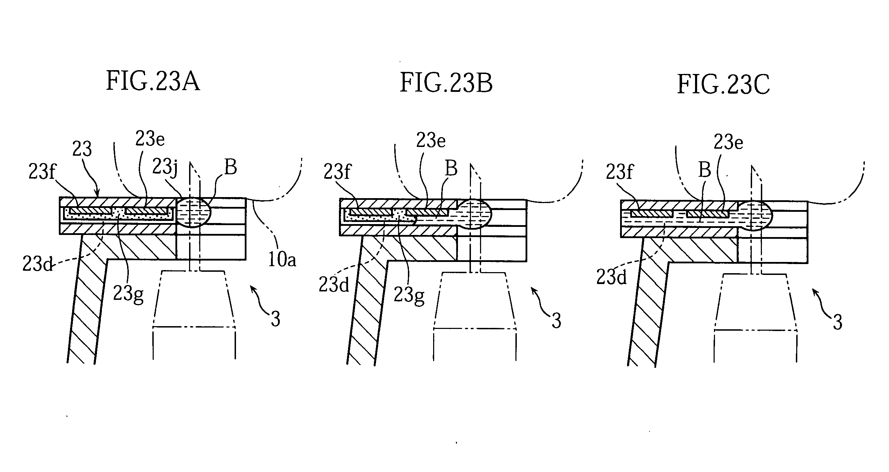

As shown in Fig. 23A, the blood B coming out from the lancing target portion 10a reaches the opening 23j of the biosensor 23. Since a slight gap is defined between the lancing needle 20a and the opening 23j of the biosensor 23, the blood coming out from the skin is properly introduced into the opening 23j without being blocked by the lancing needle 20a. As shown in Figs. 23A-23C, in the biosensor 23, the blood B moves in the capillary 23d by the capillary force generated in the capillary 23d. In this movement, the blood B dissolves the reagent portion 23g, whereby a liquid phase reaction system is established in the capillary 23d. In the liquid phase reaction system, electrons are taken out from a particular component such as glucose in the blood, and the electrons are supplied to the working electrode 23e. In the apparatus body 3, a voltage is applied between the working electrode 23e and the counter electrode 23f through the terminals 35 (See Fig. 17), and the amount of electrons moved to the working electrode 23e is measured as the electric current by the terminals 35 (See Fig. 17) . Based on the current measurements, the analysis of the particular component, e. g. the calculation of the glucose level is performed in the apparatus body 3.

After the analysis of the particular component, the used lancet 2 is removed. The projections 22B of the lancet 2 are disengaged from the stepped portion 55 of the lancing mechanism 5, so that the pressing force exerted to the casing 22 of the lancet 2 is removed. Therefore, the casing 22 returns to its original configuration so that the lancet body 20 comes into contact with the projections 22Aa and 22Ab of the casing 22 again, whereby the lancet body 20 is fixed to the casing 22. Therefore, in removing the lancet 2, the casing 22 and the lancet body 20 are combined as one piece, and the lancing needle does not project from the casing 22, which provides safety. Since such advantage is obtained without attaching the cap 21 of the lancet 2 again, the trouble of attaching the cap 21 again can be saved. Moreover, the user need not worry that he or she might forget to attach the cap 21. Further, the casing 22 and the lancet body 20 need not be removed individually, and the lancet body 20 can be removed in removing the casing 22. Therefore, the removal of the lancet 2 is easy.

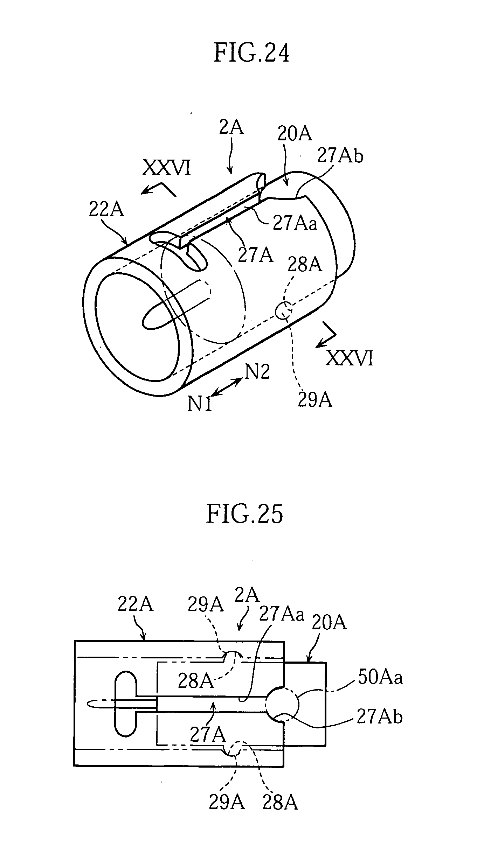

A second embodiment of the present invention will be described below with reference to Figs. 24-27.

Similarly to the foregoing lancet 2 (See Figs. 9-11), the lancet 2A shown in Figs. 24-26 includes a lancet body 20A and a casing 22A. Although the parts corresponding to the cap 21 and the biosensor 23 of the foregoing lancet 2 are not illustrated in the figures, the lancet 2A may also include a cap and a biosensor.

The casing 22A includes a slit 27A, and recesses 28A as a contact portion. The slit 27A is provided to allow the movement of a projection 50Aa provided at the housing 50A. The slit 27A comprises an arcuate cutout portion 27Aa and a linear portion 27Ab to be generally T-shaped as a whole. In the casing 22, the arcuate cutout portion 27Aa and portions on opposite sides of the linear portion 27Ab function as a leaf spring. The width of the linear portion 27Ab is smaller than the diameter of the projection 50Aa of the housing 50A so that the width can be enlarged by the projection 50Aa of the housing 50A. The recesses 28A of the casing 22A engage projections 29A provided at the lancet body 20A when the width of the linear portion 27Ab of the slit 27A is not enlarged.

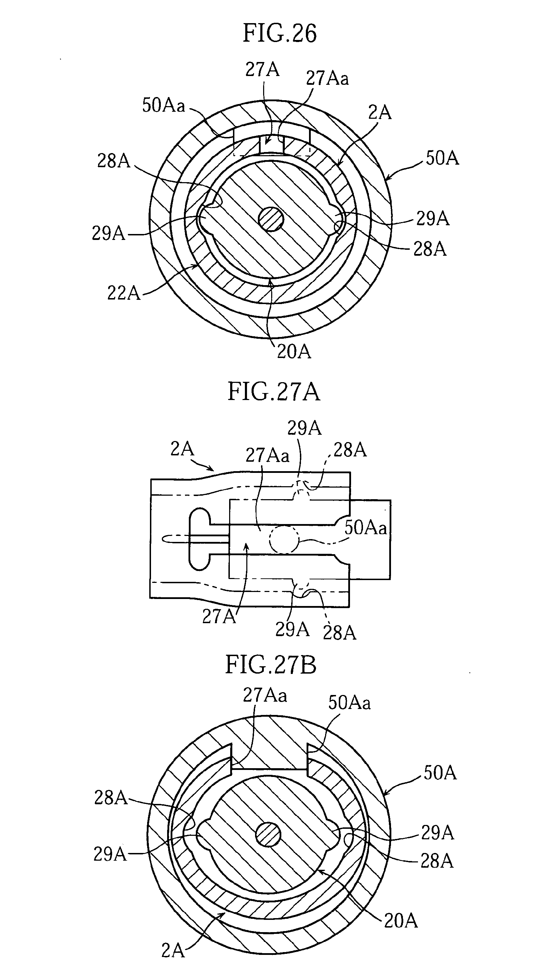

In the lancet 2A, when the projection 50Aa of the housing 50A is not positioned at the linear portion 27Ab of the slit 27A, the width of the linear portion 27Ab of the slit 27A is not enlarged. Therefore, the projections 29A of the lancet body 20A are held in engagement with the recesses 28A of the casing 22, whereby the lancet body 20A is held by the casing 22A. As better shown in Figs. 27A and 27B, when the projection 50Aa of the housing 50A is positioned at the linear portion 27Ab of the slit 27A, the width of the linear portion 27Ab of the slit 27A is enlarged, whereby the projections 29A of the lancet body 20A are disengaged from the recesses 28A of the casing 22A. In this state, the lancet body 20A is movable relative to the casing 22A.

Although the contact portion is provided as the recesses 29A in this embodiment, the contact portion may comprise a projection similarly to the first embodiment. Conversely, the contact portion in the first embodiment may comprise a recess similarly to this embodiment.

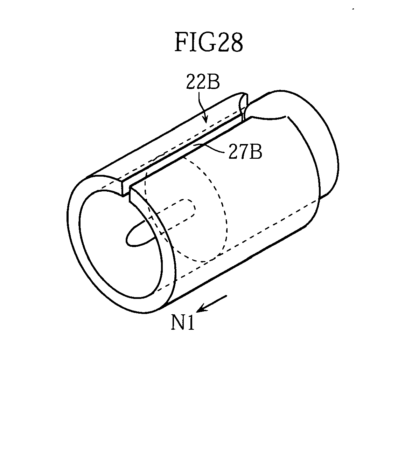

The lancet may be designed as shown in Figs. 28, 29 and 30, and 31A-31C.

In the lancet shown in Fig. 28, the casing 22B includes a slit 27B which extends up to the end on the arrow N1 side.

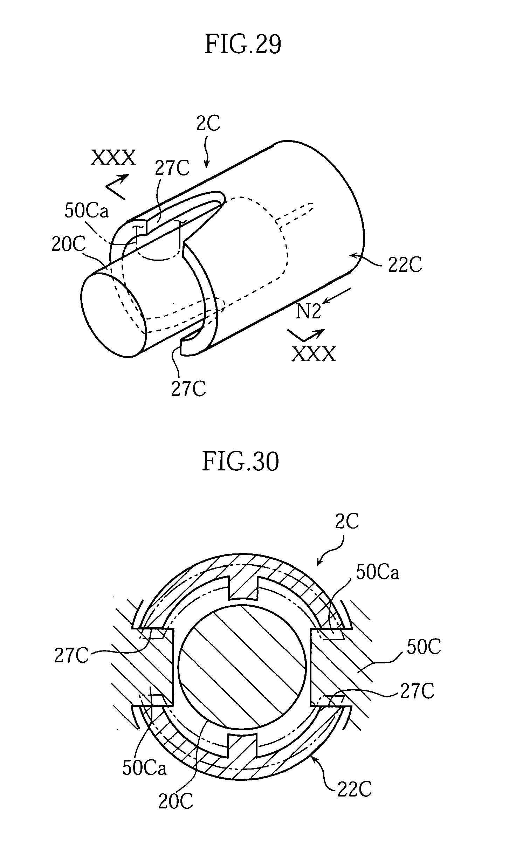

In the lancet 2C shown in Figs. 29 and 30, the casing 22C includes a pair of cutouts 27C each of which gradually widens as it extends toward the end on the arrow N2 side. In the lancet 2C, when projections 50Ca of the housing 50C are not positioned in the cutouts 27C, the lancet body 20C is held by the casing 22C. When the projections 50Ca are positioned in the cutouts 27C, the width of the cutouts 27C is enlarged, so that the lancet body 20C is movable relative to the casing 22C.

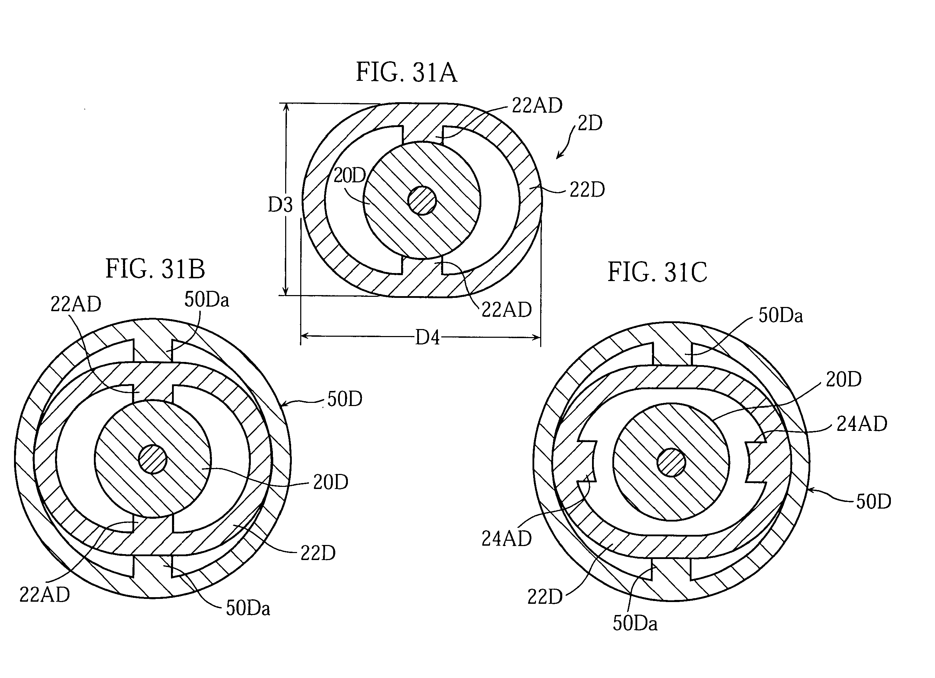

In the lancet 2D shown in Fig. 31A, the casing 22D has an oval cross section. Specifically, the outer diameter D4 of the casing 22D at a portion which is not formed with projections 22AD is larger than the outer diameter D3 at a portion formed with projections 22AD. The lancet body 20D is held by applying a pressing force from the portion with the smaller outer diameter D3 to the lancet body 20D. As shown in Fig. 31B, the lancet 2D is set in the housing 50D while aligning the projections 22AD of the casing 22D with the projections 50Da of the housing 50. In this state, the cross sectional shape of the casing 22D does not change and is maintained in its natural state. Thus, the holding of the lancet body 20D by the casing 22D is maintained. As shown in Fig. 31C, when the casing 22D is rotated through 90 degrees relative to the housing 50D, the projections 50Da of the housing 50D come into contact with the portion of the casing 22D with the larger outer diameter D4 (the portion which is not formed with the projections 22AD) . As a result, the diameter of the portion which is not formed with the projections 22AD reduces, whereas the diameter of the portion which is formed with the projections 22AD increases. As a result, the projections 22AD of the casing 22D separate from the surface of the lancet body 20D, so that the lancet body 20D is movable relative to the casing 22D.

The rotation of the casing 22D relative to the housing 50D may be performed by providing an operating portion (e. g. projection) at the casing 22D and operating the operating portion by the user to rotate the casing 22D relative to the housing 50D. Alternatively, a mechanism for rotating the casing 22D may be provided in the lancing apparatus or the casing 22D may be so designed as to rotate in inserting the casing 22D into the housing 50D.

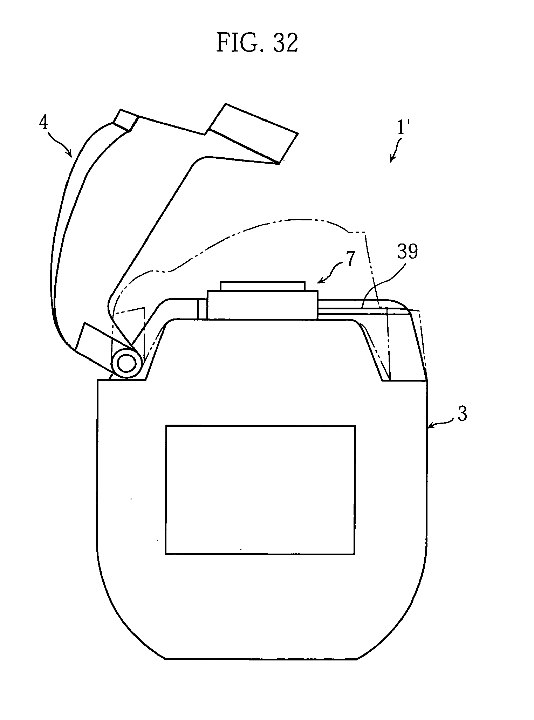

Next, a third embodiment of the present invention will be described below with reference to Figs. 32-42. In these figures, the elements which are identical or similar to those of the first embodiment are designated by the same reference signs as those used for the first embodiment, and overlapping description thereof will be omitted.

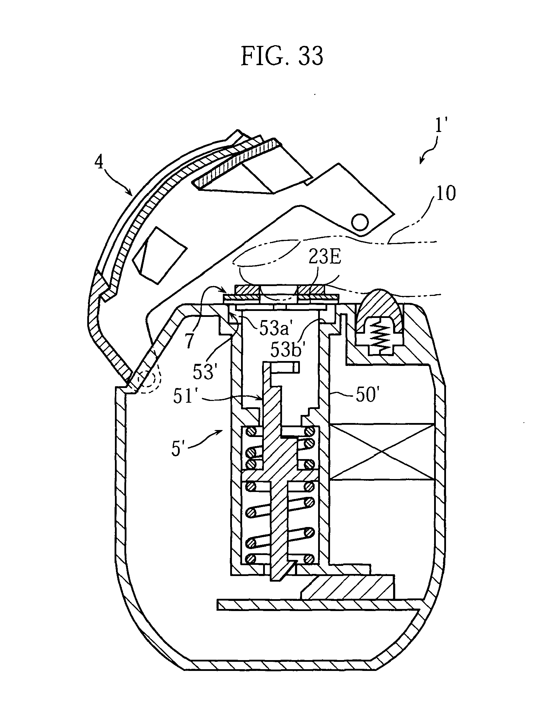

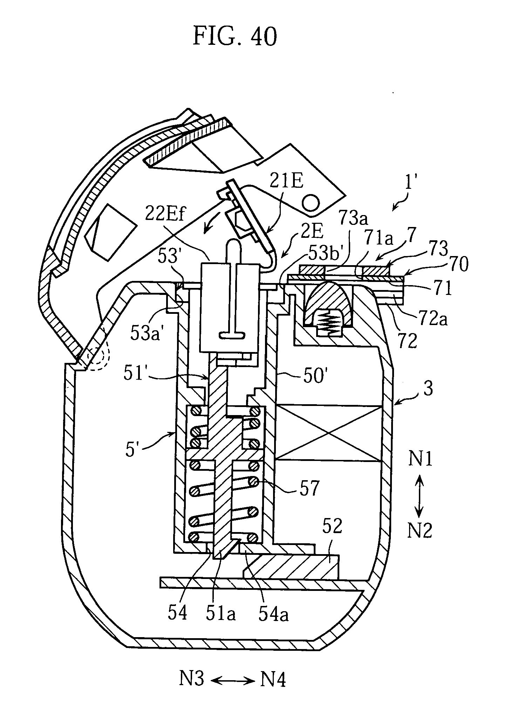

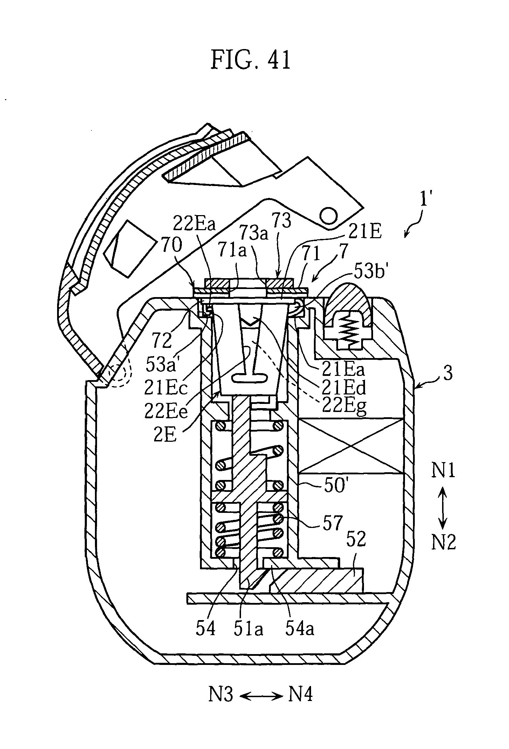

Similarly to the lancing apparatus 1 (See Figs. 1-3) of the first embodiment of the present invention, the lancing apparatus 1' shown in Figs. 32 and 33 is used with a lancet 2E attached thereto (See Figs. 40 and 41) . The lancing apparatus 1' includes a lancing mechanism 5' and a lid closing mechanism 7.

As shown in Fig. 2; although the principle of operation of the lancing mechanism 5' is similar to that of the lancing mechanism 5 (See Figs. 2 and 22) of the lancing apparatus 1 (See Figs. 1-3), the structure of the housing 50' and the lancet holder 51' differs from that of the foregoing lancing mechanism.

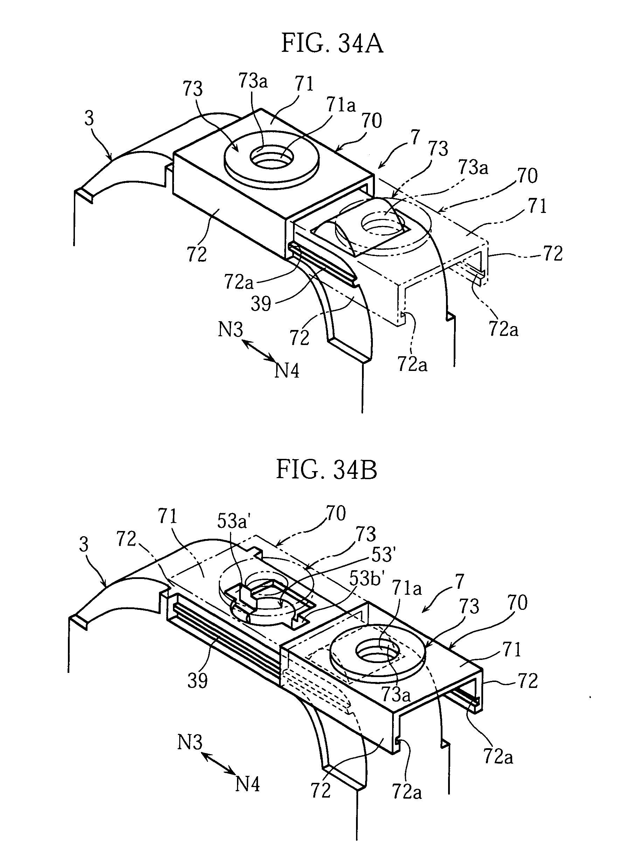

As shown in Figs. 33 and 34B, the housing 50' includes an opening 53' for inserting the lancet 2E. The opening 53' includes cutouts 53a' and 53b'. As shown in Fig. 41, the cutout 53a' serves to receive a hook 21Ec and a projection 22Ea of the lancet 2E, which will be described later. The cutout 53b' serves to receive a connection portion 21Ea of the lancet 2E, which will be described later.

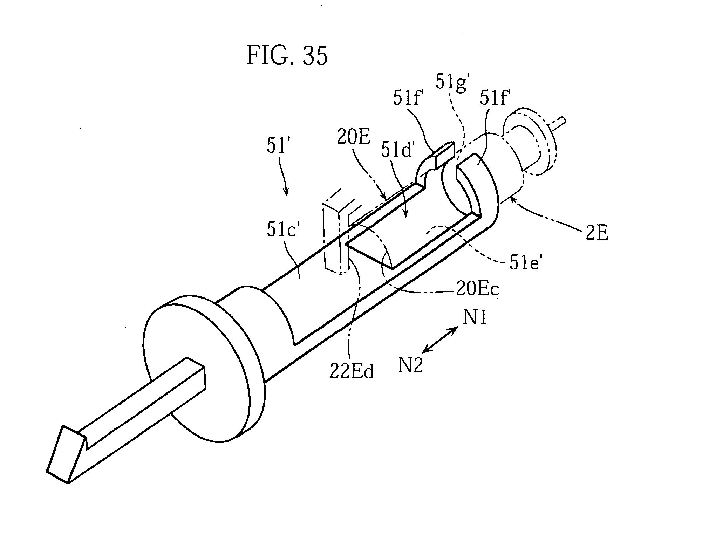

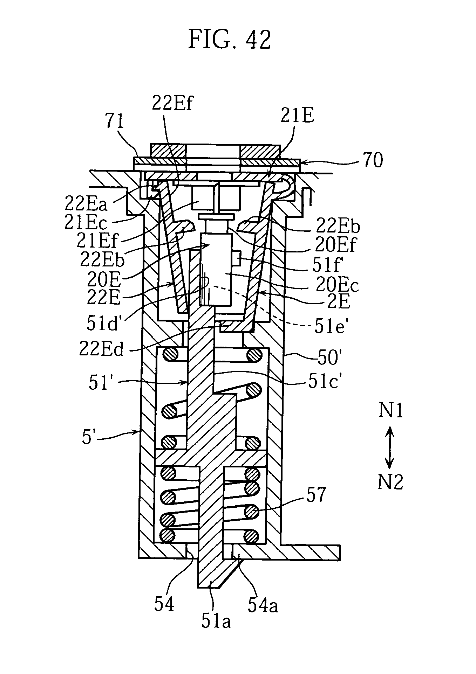

As shown in Figs. 35 and 42, the lancet holder 51' serves to hold the lancet 2E. The lancet holder 51' includes an opening portion 51c' and an accommodation portion 51d'. The opening portion 51c' is provided to avoid the interference with a stopper portion 22Ed of the lancet 2E, which will be described later, when the lancet holder 51' is moved in the directions of arrows N1 and N2. The accommodation portion 51d' serves to hold the lancet body 20E of the lancet 2E and includes a space 51e' for embedding part of a holding portion 20Ec of the lancet body 20E, and a pair of spring portions 51f'. The paired spring portions 51f' serve to apply a pressing force to the lancet body 20E to firmly hold the lancet body 20E in the accommodation portion 51d' . The respective ends of spring portions 51f' face each other via a gap 51g' . The gap 51' serves to provide the spring portions 51f' with spring properties and prevent the interference with the stopper portion 22Ed of the lancet 2E.

As shown in Figs. 40 and 41, the lid closing mechanism 7 is provided for closing the lid 21E of the lancet 2E when the lancet 2E is accommodated in the housing 50'. The lid closing mechanism 7 includes a slider 70.

As shown in Figs. 34A and 34B, the slider 70 is movable in the directions of arrows N3 and N4 relative to the apparatus body 3 and can be selectively located at a position above the opening 53' of the housing 50' or a position avoiding the opening 53'. The slider 70 includes an engagement portion 71 and a guide portion 72.

When the slider 70 is moved in the direction of N3 to close the lid 21E of the lancet 2E, the engagement portion 71 comes into contact with the lid 21E. The engagement portion is spaced from the upper surface of the apparatus body 3 by a predetermined distance (See Figs. 40 and 41). The engagement portion 71 is formed with a through-hole 71a. The through-hole 71a is provided for exposing part of the biosensor 23E to allow the fingertip 10 to come into contact with the biosensor 23E (See Fig. 33) or for allowing the movement of the lancing needle 20Ea of the lancet 2E, which will be described later. To the engagement portion 71, a contact member 73 is attached. Similarly to the contact member 56 of the first embodiment, the contact member 73 is brought into close contact with the fingertip 10 when the fingertip 10 is inserted into the accommodation space 11. The contact member 73 is formed with a through-hole 73a which has the same configuration as that of the through-hole 71a of the slider 70. To ensure the close contact with the fingertip 10 (See Fig. 4), the contact member 73 is made of an elastic body such as rubber or a foamed material.

The guide portion 72 defines the movement path of the engagement portion 71 (slider 70) and includes grooves 72a extending in the directions of arrows N3 and N4. The grooves 72a engage projections 39 provided at the apparatus body 3 and extending in the directions of arrows N3 and N4. Thus, with the projections 39 held in engagement with the grooves 72a, the slider 70 is movable in the directions of arrows N3 and N4 relative to the apparatus body.

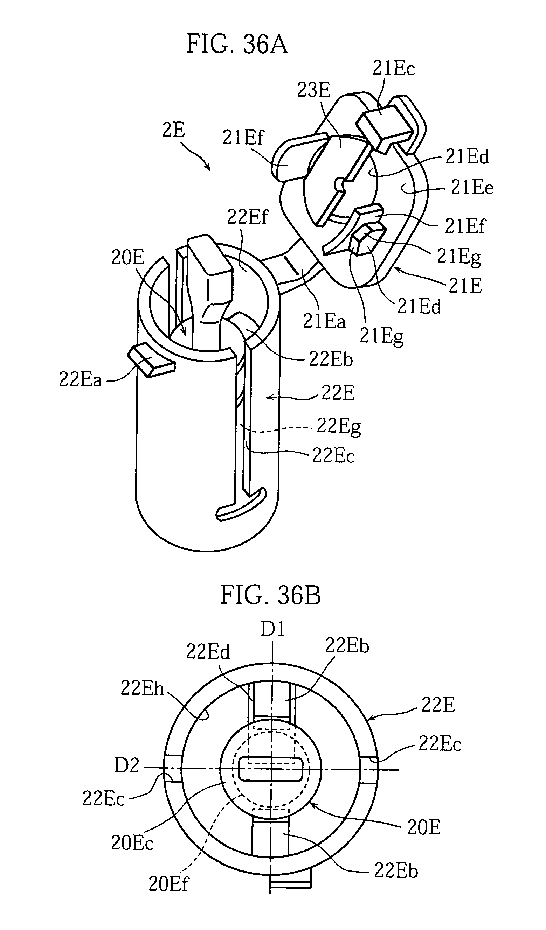

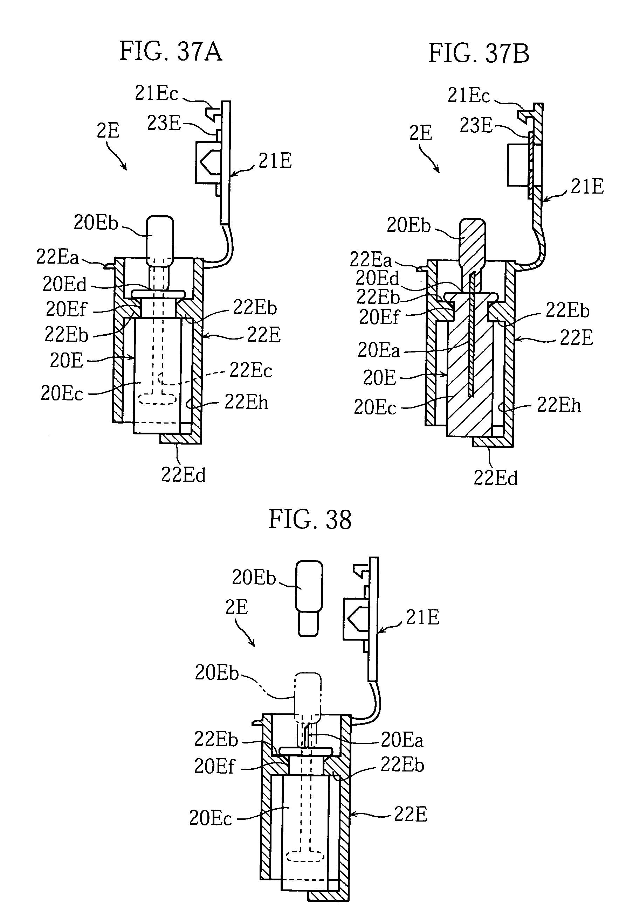

As shown in Figs. 36A, 37A and 37B, the lancet 2E is inserted, in use, into the housing 50' of the apparatus body 3 (See Figs. 40 and 41). The lancet is made up of the lancet body 20E, the lid 21E, the casing 22E and the biosensor 23E. In the lancet 2E, the lancet body 20E is held in the casing 22E when an external force is not applied to the casing 22E . When an intended external force is applied to the casing 22E, the lancet body 20E becomes movable relative to the casing 22E.

As shown in Figs. 37A and 37B, the lancet body 20E is provided with the lancing needle 20Ea, a protector 20Eb, the holding portion 20Ec and a cutout 20Ed. The protector 20Eb covers the tip end of the lancing needle 20Ea and is removable by the user to expose the tip end of the lancing needle 20Ea, as shown in Fig. 38. The holding portion 20Ec is a portion which is held by the lancet holder 51' as shown in Fig. 35 and in which the opposite end of the lancing needle 20Ea from the tip end is embedded as shown in Fig. 37B. As shown in Figs. 37A, 37B and 38, the holding portion 20Ec is formed with an annular recess 20Ef. The recess 20Ef serves to receive a pair of engagement projections 22Eb of the casing 22E, which will be described later. In this way, when an external force is not applied to the casing 22E, the lancet body 20E is held in the casing 22E. The cutout 20Ed is provided between the protector 20Eb and the holding portion 20Ec to facilitate the separation between the protector 20Eb and the holding portion 20Ec.

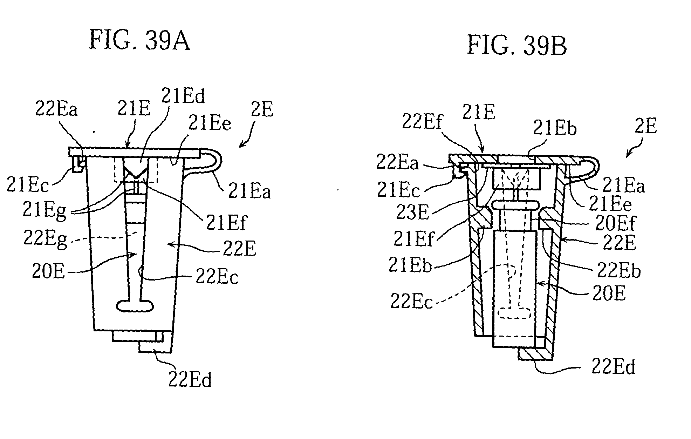

As shown in Figs. 36A, 39A and 39B, the lid 21E serves to close the upper opening 22Ef of the casing 22E and apply an external force to the casing 22E to make the lancet body 20E movable relative to the casing 22E. The lid 21E is attached to the casing 22E via the connection portion 21Ea and is provided with a through-hole 21Eb, the hook 21Ec and a pair of operative portions 21Ed.

The through-hole 21Eb serves to expose part of the biosensor 23E and allow the fingertip 10 to come into contact with the biosensor 23E in lancing (See Fig. 33). The hook 21Ec is brought into engagement with the projection 22Ea of the casing 22E, which will be described later, to maintain the closed state of the opening 22Ef of the casing 22E with the lid 21E. The paired operative portions 21Ed serve to enlarge the width of the slit 22Ec of the casing 22E (gap 22Eg) by coming into engagement with the slits 22Ec, which will be described later. Specifically, insertion pieces 21Ef are so provided as to project from a surface 21Ee of the lid 21E in the thickness direction of the lid 21E, and the operative portions 21Ed are provided on the insertion pieces to project toward the periphery of the lid 21E. Each of the operative portions 21Ed includes two inclined surfaces 21Eg to be tapered. Therefore, the operative portions 21Ed can be easily and reliably inserted into the slits 22Ec of the casing 22E.

As shown in Figs. 36A, 36B and 37A, the casing 22E holds the lancet body 20E and is cylindrical as a whole. The casing 22E is provided with the projection 22Ea, the paired engagement projections 22Eb, the paired slits 22Ec and the stopper portion 22Ed.

As shown in Figs. 39A and 39B, the projection 22Ea serves to engage the hook 21Ec of the lid 21E.

As shown in Figs. 36B, 37A and 37B, the paired engagement projections 22Eb engage the recess 20Ef of the holding portion 20Ec of the lancet body 20E. The engagement projections 22Eb project from the inner surface 22Eh of the casing 22E so as to face each other on the first diameter axis D1.

As shown in Figs. 36A, 36B, 39A and 39B, the paired slits 22Ec are provided to allow the change of the cross-sectional shape of the casing 22E. The slits 22Ec extend vertically and face each other on the second diameter axis D2.

As shown in Figs. 36B and 39B, the stopper portion 22Ed prevents the lancet body 20E from dropping from the casing 22E. The stopper portion 22Ed extends toward the center of the casing 22E along the first diameter axis D1.

The biosensor 23E shown in Fig. 36A is designed to move blood by capillary force and measure the concentration of a particular component (e.g. glucose) in blood by an electrode method. The biosensor 23E is bonded to the surface 21Ee of the lid 21E. Part of the biosensor 23E is exposed at the opposite surface of the lid 21E from the surface 21Ee through the through-hole 21Eb of the lid 21E. Thus, the through-hole 21Eb of the lid 21E is partially closed by the biosensor 23E, and a sufficient space for allowing the lancet body 20E to pass through the through-hole 21Eb of the lid 21E is not provided. Though not illustrated, the biosensor 23E has a structure which is similar to that of the biosensor 23 (See Figs. 17-20) described in the first embodiment of the present invention.

The use and operation of the lancing apparatus 1' will be described below. Since the lancing, operation and the computing (analysis) operation of the lancing apparatus 1 are similar to those of the lancing apparatus 1 (See Figs. 1-3) of the first embodiment described before, the detailed description of the lancing operation and the computing (analysis) operation will be omitted.

To perform the lancing operation using the lancing apparatus 1', with the lid 4 opened as shown in Figs. 32 and 33, the opening 53' of the apparatus body 3 is exposed as shown in Fig. 34B. The opening 53' can be exposed by moving the slider 70 in the direction of arrow N4 by the user.

Subsequently, as shown in Fig. 40, with the projection 22Ea and the connection portion 21Ea of the lancet 2E positioned at the cutouts 53a' and 53b', respectively, the lancet 2E is inserted into the housing 50' through the opening 53', with the lid 21E opened. As shown in Figs. 37A and 37B, in the lancet 2E in this state, the engagement projections 22Eb of the casing 22E are received in the recess 20Ef of the lancet body 20E so that the lancet body 20E is fixed to the casing 22E.

Subsequently, the protector 20Eb of the lancet body 20E is removed as shown in Fig. 38, and then the slider 70 is moved in the direction of arrow N3 as shown in Fig. 34A. As a result, as shown in Figs. 39A, 39B and 40-42, the lid 21E of the lancet 2E is closed. Specifically, when the slider 70 is moved in the direction of arrow N3, the engagement portion 71 of the slider 70 comes into contact with the lid 21E to apply a force in the direction of arrow N3 to the lid 21E. As a result, the lid 21E moves in the direction to close the upper opening 22Ef of the casing 22E. When the slider 70 is further moved in the direction of arrow N3, the engagement portion 71 comes to a position directly above the opening 53', and the lid 21E is caused to get under the engagement portion 71.

As shown in Figs. 41 and 42, in the process of causing the lid 21E to get under the engagement portion 71, a downward force is exerted to the entirety of the lancet 2E. Therefore, the holding portion 20Ec of the lancet body 20E is fitted into the accommodation portion 51d' of the lancet holder 51' so that the lancet body 20E becomes movable together with the lancet holder 51' . Further, in inserting the lancet 2E into the housing 50', the lancet holder 51' can be moved in the direction of arrow N2 to bring the engagement portion 51a of the lancet holder 51' into engagement with the periphery 54a of the through-hole 54 of the housing 50' . By this operation, the coil spring 57 is compressed and stores an elastic restoring force.

The holding portion 20Ec of the lancet body 20E may be fitted into the accommodation portion 51' of the lancet holder 51' in inserting the lancet 2E into the housing 50'. The engagement portion 51a of the lancet holder 51' may be brought into engagement with the periphery 54a of the through-hole 54 separately from the operation to attach the lancet 2E. Specifically, the engagement portion 51a may be brought into engagement with the periphery 54a of the through-hole 54 before the lancet 2E is attached to the lancet holder 51'.

In the process of causing the lid 21E to get under the engagement portion 71, the insertion pieces 21Ef of the lid 21E are received in the accommodation portion 22E of the lancet 2E, while the operative portions 21Ed enter the slits 22Ec. As a result, the gaps 22Eg of the slits 22Ec are enlarged, whereby the engagement projections 22Eb are disengaged from the recess 20Ef of the lancet body 20E. Therefore, the lancet body 20E is movable relative to the casing 22E. Finally, the hook 21Ec of the lid 21E engages the projection 22Ea of the casing 22E, whereby the state in which the upper opening 22Ef of the casing 22E is closed by the lid 21E is maintained.

In this way, in the lancing apparatus 1', the preparation for the lancing can be completed just by moving the slider 70 in the direction of arrow N3 after the lancet 2E is set in the housing 50'. Therefore, the preparation for the lancing in the lancing apparatus 1' is extremely easy.

Subsequently, the fingertip 10 is placed on the apparatus body 3 so as to close the through-hole 73a of the contact member 73 (See Fig. 33), the lid 4 is closed, and then the lancing operation is performed. Similarly to the lancing apparatus 1' (See Figs. 1-3), the lancing operation is performed by moving the lancet body 20E together with the lancet holder 51' in the direction of arrow N1 to cause the lancing needle 20a to stick into the fingertip 10 (See Figs. 22A-22C). The movement of the lancet holder 51' may be performed by moving the operative portion 52 so that the operative portion 52 comes into engagement with the engagement portion 51a.

In the lancing apparatus 1', the blood extracted from the fingertip 10 is supplied to the biosensor 2 3E (See Figs. 23A-23C). Using the biosensor 23E, the analysis of a particular component (e.g. computation of the glucose level) is performed.

After the analysis of a particular component, the lancet 2E after use is removed. The removal of the lancet 2E is performed, with the opening 53' exposed by moving the slider 70 in the direction of arrow N4. The casing 22E of the lancet 2E is provided with the stopper portion for preventing the lancet body 20E from dropping from the casing 22E. Further, the upper opening 22Ef of the casing 22E is closed by the lid 21E, and as described above, the through-hole 21Eb of the lid 21E does not include the space for allowing the lancet body 20E to pass therethrough. Therefore, after the lancet 2E is used, the lancet body 20E does not drop from the casing 22E, and the removal of the used lancet 2E from the housing 50' and the disposal of the removed lancet 2E can be performed safely and hygienically.

Although the lancet is provided with the lid in the third embodiment of the present invention, a member corresponding to the lid of the lancet may be provided at the apparatus body of the lancing apparatus.

## Claims

1. A lancet comprising a lancet body provided with a lancing element, and a casing including a space extending throughout the casing for retaining the lancet body therein,
wherein the lancet body is fixed to the casing when an external force exceeding a predetermined level in a particular direction is not applied to the casing, whereas the lancet body becomes movable relative to the casing when an external force exceeding the predetermined level in the particular direction is applied to the casing.

2. The lancet according to claim 1, wherein the particular direction crosses a direction in which the space extends.

3. The lancet according to claim 1, wherein the casing has a cross-sectional shape which changes when the external force exceeding the predetermined level in the particular direction is applied to the casing; and
wherein the lancet body becomes movable relative to the casing when the cross-sectional shape of the casing is changed.

4. The lancet according to claim 3, wherein the casing includes a contact portion which comes into contact with the lancet body when the external force exceeding the predetermined level in the particular direction is not applied to the casing; and
wherein a gap is defined between the contact portion and the lancet body when the cross-sectional shape of the casing is changed.

5. The lancet according to claim 4, wherein the contact portion comprises a projection.

6. The lancet according to claim 4, wherein the contact portion comprises a recess, and
wherein the lancet body is provided with a projection for coming into engagement with the recess.

7. The lancet according to claim 4, wherein an outer diameter of the casing at a portion where the contact portion is not provided is larger than an outer diameter of the casing at a portion where the contact portion is provided.

8. The lancet according to claim 1, wherein the casing is formed with a projection for actively causing the external force in the particular direction to be applied to the casing.

9. The lancet according to claim 3, wherein the casing is formed with an opening for allowing the cross-sectional shape of the casing to change when the external force in the particular direction is applied.

10. The lancet according to claim 9, wherein the opening comprises a cutout or a slit.

11. The lancet according to claim 3, wherein, after the cross-sectional shape of the casing is changed, the cross-sectional shape returns to an original shape when the application of the external force to the casing in the particular direction is removed.

12. The lancet according to claim 1, further comprising a lid for selectively opening or closing an upper opening of the casing.

13. The lancet according to claim 12, wherein the lid is attached to the casing.

14. The lancet according to claim 12, wherein, when the lid closes the upper opening of the casing, the external force exceeding the predetermined level in the particular direction is applied to the casing to change the cross-sectional shape of the casing; and
wherein the lancet body becomes movable relative to the casing when the cross-sectional shape of the casing is changed.

15. The lancet according to claim 14, wherein the casing is formed with an opening for allowing the cross-sectional shape of the casing to change when the external force in the particular direction is applied; and
wherein the lid includes an operative portion which comes into engagement with the opening and applies the external force in the particular direction to the casing when the lid closes the upper opening.

16. The lancet according to claim 14, wherein the casing includes a stopper portion for preventing the lancet body from dropping through a lower opening of the casing when the lancet body is movable relative to the casing.

17. The lancet according to claim 13, further comprising a fixer for fixing the lid to the casing when the upper opening is closed by the lid.

18. The lancet according to claim 17, wherein the fixer comprises a projection provided at one of the lid and the casing, and a hook provided at the other one of the lid and the casing for engagement with the projection.

19. The lancet according to claim 1, wherein the casing holds an analytical tool for analyzing a particular component contained in body fluid extracted from a lancing target portion.

20. The lancet according to claim 19, wherein the analytical tool includes a capillary for moving blood by capillary force, a through-hole for allowing movement of the lancing element, and an introduction port which communicates with the through-hole for introducing blood to the capillary.

21. The lancet according to claim 14, wherein the lid holds an analytical tool for analyzing a particular component contained in body fluid extracted from a lancing target portion.

22. The lancet according to claim 21, wherein the analytical tool includes a capillary for moving blood by capillary force, a through-hole for allowing movement of the lancing element, and an introduction port communicating with the through-hole for introducing blood to the capillary.

23. A lancing apparatus used with a lancet attached to the lancing apparatus,
wherein the lancet includes a lancet body provided with a lancing element, and a casing formed with a space extending throughout the casing for retaining the lancet body therein, the lancet body being fixed to the casing when an external force exceeding a predetermined level in a particular direction is not applied to the casing, the lancet body being movable relative to the casing when an external force exceeding the predetermined level in the particular direction is applied to the casing,
wherein the lancing apparatus is configured to apply an external force to the casing to make the lancet body movable relative to the casing when the lancet is attached.

24. The lancing apparatus according to claim 23, comprising an operative portion for coming into engagement with the casing to apply the external force to the casing.

25. The lancing apparatus according to claim 24, wherein the casing of the lancet is formed with a projection for actively causing the external force in the particular direction to be exerted to the casing,
wherein the operative portion is configured to come into engagement with the projection to apply the external force to the casing when the lancet is attached.

26. The lancing apparatus according to claim 24, wherein the casing of the lancet is formed with an opening for allowing a cross-sectional shape of the casing to change when the external force in the particular direction is applied to the casing,
wherein the operative portion is configured to come into engagement with the opening to change the cross-sectional shape of the casing when the lancet is attached to the lancing apparatus.

27. A lancing apparatus used with a lancet attached to the lancing apparatus,
wherein the lancet comprises a lancet body provided with a lancing element, a casing formed with a space extending throughout the casing for retaining the lancet body therein and a lid for closing an upper opening of the casing, the lancet body being fixed to the casing when an external force exceeding a predetermined level in a particular direction is not applied to the casing, the lancet body being movable relative to the casing when an external force exceeding the predetermined level in the particular direction is applied to the casing by closing the upper opening by the lid,
wherein the apparatus comprises external force applying means for applying an external force to the lid to close the upper opening by the lid in a state in which the lancet is attached.

28. The lancing apparatus according to claim 27, further comprising an accommodation space for accommodating the lancet,
wherein the external force applying means includes a slider which is reciprocally movable in a direction crossing a direction in which the accommodation space extends in a state in which the lancet is accommodated in the accommodation space, and
wherein the slider includes an engagement portion for coming into engagement with the lid.

29. The lancing apparatus according to claim 28, wherein the engagement portion is reciprocally movable between a first position directly above the accommodation space and a second position avoiding a position directly above the accommodation space, and wherein the engagement portion, in moving from the second position to the first position, comes into engagement with the lid to apply an external force to the lid to cause the lid to close an upper opening of the casing.

30. The lancing apparatus according to claim 27, wherein the casing of the lancet includes a stopper portion for preventing the lancet body from dropping through a lower opening of the casing in a state in which the lancet body is movable relative to the casing,
wherein the lancing apparatus further comprises a lancet holder for holding the lancet body,
wherein the lancet holder is movable without interfering with the stopper portion in a state in which the lancet is attached.

31. The lancing apparatus according to claim 30, wherein the lancet holder is formed with a recess for avoiding the interference with the stopper portion.
